# EUROPEAN PATENT APPLICATION

(11) **EP 3 058 869 A1**
(43) Date of publication of application: **24.08.2016**
(21) Application number: 16155725.1
(22) Date of filing: 15.02.2016
(51) Int. Cl.: A61B 5/021, A61G 7/05, A61G 7/16, A61B 5/024, A61B 5/145, A61M 21/00, A61B 5/16, A47C 20/04, A61B 5/00, A61B 5/11, G01G 19/44

(54) **MONITORING A PATIENT'S STATE TO CONTROL THE PATIENT SUPPORT**

(30) Priority: 18.02.2015 US 201562117913 P
(71) Applicant: Allen Medical Systems, Inc., Batesville, IN 47006 (US)
(72) Inventor: DRAKE, Jesse S, Westborough, MA Massachusetts 01581 (US)
(74) Representative: Findlay, Alice Rosemary

(57) **Abstract**

A person support apparatus can be adjusted to support a person in a number of different positions, including a laying-down position, a seated position, and positions intermediate the laying-down and seated positions. One or more sensors, which may be carried by, worn by or attached to the person, the person support apparatus, or another device, can be used to detect physiological responses of the person. A computing device, such as a bed controller, can generate an indication of the person's state based on one or more of the sensor inputs. The computing device can, based on the patient sate indication, cause an adjustment to the position or configuration of the person support apparatus to occur.

## Description

A person support apparatus, such as a stretcher, a hospital bed or a similar device, may be used to support a person in a number of different positions, including a laying-down position and/or a seated position. Such a product may be found, for example, in healthcare facilities, homes, and/or other locations in which patient care is provided. The person support apparatus may include actuators that enable the position or configuration of the person support apparatus to be adjusted. A control unit can be used to control these adjustments. Sensors can be used to obtain patient physiological data.

According to at least one aspect of this disclosure, a person support apparatus control system includes one or more computing devices configured to: determine a patient lucidity state based on a sensor input; determine a current angular position of a support section of a person support apparatus; and cause the support section of the person support apparatus to move from the current angular position to a new angular position in response to the patient lucidity state.

In one embodiment the sensor input includes data indicative of the patient's blood pressure, and the control system is configured to compute the patient lucidity state based on the data indicative of the patient's blood pressure and cause the support section of the person support apparatus to move from the current angular position to the new angular position in response to the patient lucidity state computed based on the data indicative of the patient's blood pressure.

In another embodiment the sensor input includes data indicative of the patient's blood oxygen saturation level, and the control system is configured to compute the patient lucidity state based on the data indicative of the patient's blood oxygen saturation level and cause the support section of the person support apparatus to move from the current angular position to the new angular position in response to the patient lucidity state computed based on the data indicative of the patient's blood oxygen saturation level. The sensor input may include data indicative of the patient's heart rate, and the control system is configured to compute the patient lucidity state based on the data indicative of the heart rate and cause the support section of the person support apparatus to move from the current angular position to the new angular position in response to the patient lucidity state computed based on the data indicative of the patient's heart rate. The sensor input may include data indicative of at least two of: the patient's heart rate, the patient's blood oxygen saturation level, and the patient's blood pressure; the control system is configured to compute the patient lucidity state based on a combination of at least two of the patient's heart rate, the patient's blood oxygen saturation level, and the patient's blood pressure; and the control system is configured to cause the support section of the person support apparatus to move from the current angular position to the new angular position in response to the patient lucidity state computed based on the data indicative of at least two of the patient's heart rate, the patient's blood oxygen saturation level, and the patient's blood pressure. The system may be configured to compare the patient lucidity state to a previously-determined patient lucidity state and move the support section of the patient support apparatus to an inclined position if the comparison of the patient lucidity state to the previously-determined patient lucidity state is indicative of an increase in the patient's lucidity.

The system may be configured to compare the patient lucidity state to a previously-determined patient lucidity state and move the support section of the patient support apparatus toward a flat position if the comparison of the patient lucidity state to the previously-determined patient lucidity state is indicative of a decrease in the patient's lucidity.

In another aspect, a person support apparatus has articulating head and foot sections, the patient support apparatus configured to: raise the head section in response to patient lucidity data indicative of an increase in patient lucidity; and lower the head section in response to patient lucidity data indicative of a decrease in patient lucidity. The apparatus may be configured to lower the foot section in response to the patient lucidity data indicative of an increase in patient lucidity. It may be configured to cooperatively lower the foot section and raise the head section in response to the patient lucidity data indicative of an increase in patient lucidity. It may be configured to raise the foot section in response to the patient lucidity data indicative of a decrease in patient lucidity, preferably to cooperatively raise the foot section and lower the head section in response to the patient lucidity data indicative of a decrease in patient lucidity. The apparatus may include a lift mechanism to adjust the vertical height of the person support apparatus, where the person support apparatus is configured to decrease the vertical height of the person support apparatus in response to the patient lucidity data indicative of an increase in patient lucidity. Preferably it is configured to cooperatively lower the foot section, raise the head section, and lower the vertical height of the patient support apparatus in response to the patient lucidity data indicative of an increase in patient lucidity. The apparatus may include a lift mechanism to adjust the vertical height of the person support apparatus, where the person support apparatus is configured to increase the vertical height of the person support apparatus in response to the patient lucidity data indicative of a decrease in patient lucidity. Preferably it is configured to cooperatively raise the foot section, lower the head section, and increase the vertical height of the patient support apparatus in response to the patient lucidity data indicative of a decrease in patient lucidity. The person support apparatus may include a siderail and be configured to raise the siderail in response to the patient lucidity data indicative of a decrease in patient lucidity. The person support apparatus may include a torso section and be configured to adjust an angle defined by the head section and the torso section in response to a change in the patient lucidity data. The person support apparatus may also be configured to adjust a first angle defined by the head section and the torso section and a second angle defined by the foot section and the torso section, in response to a change in the patient lucidity data.

In another aspect, a method is provided for adjusting the position of a person support apparatus having at least a head section and a foot section, in response to changes in patient state, the method including, with one or more computing devices, over time: obtaining a plurality of patient physiological inputs; determining a patient state based on at least two of the patient physiological inputs; detecting changes in the patient state; and progressively adjusting the angular position of the head and foot sections of the person support apparatus in accordance with the detected changes in the patient state. The method may include progressively moving the head and foot sections to a bed position as the patient state declines, and progressively moving the head and foot sections to a chair position as the patient state improves. The method may include generating an alert indicative of a change in the patient state, and transmitting the alert to another device. The method includes sending data indicative of the patient state to a medical record database.

The invention will now be further described by way of example with reference to the accompanying drawings, in which:
FIG. 1 is a simplified schematic diagram of at least one embodiment of a person support apparatus control system as disclosed herein;
FIG. 2 is a perspective view of an illustrative embodiment of a person support apparatus in which the system of FIG. 1 may be implemented;
FIG. 3 is a simplified schematic diagram of at least one embodiment of a computing environment in which the person support apparatus control system of FIG. 1 may be implemented;
FIG. 4 is a simplified schematic diagram of an operational environment of at least embodiment of the person support apparatus control system of FIG. 1;
FIG. 5 is a simplified flow diagram of at least one embodiment of a method for adjusting the person support apparatus in response to patient state, as disclosed herein;
FIG. 6 is a simplified side elevation view of an illustrative embodiment of a person support apparatus in a chair position;
FIG. 7 is a simplified side elevation view of the person support apparatus of FIG. 6 in an intermediate position; and
FIG. 8 is a simplified side elevation view of the person support apparatus of FIG. 6 in a bed position.

A person may be placed on a person support apparatus (e.g., a stretcher, ambulatory chair, integrated chair/stretcher, or hospital bed) for a variety of reasons, including before or after having undergone a medical procedure, therapy, or examination. When a person is experiencing a non-lucid state (e.g., a delirious or unconscious state, which may be the result of anesthesia or certain medications, for example), placing the person in a laying down position can be desirable for safety and/or comfort reasons. Conversely, when a patient is recovering from a non-lucid state, it is important that the patient does not stand up too quickly, lest the patient lose consciousness and be injured by a subsequent fall. In order to facilitate a safe and comfortable transition from a lucid state to a non-lucid state, or vice versa (for example, while recovering from surgery, or after being placed under the observation of a doctor), a person support apparatus control system 100 as disclosed herein adjusts the position of a person support apparatus in accordance with a measured indication of patient state.

As used herein, "patient state" may refer to, among other things, a physiological state of a person that includes a mental component, a physical component, or a combination of mental and physical components. For example, in some embodiments, "patient state" may refer to a relative degree of lucidity of the patient, e.g., a degree to which the person is conscious, able to make decisions for themselves, the patient's subjective feeling of well-being, i.e., feeling well/not well, relative degree of anxiety, mental stress, perception of pain, etc.), while in other embodiments, the patient state may refer to an actual decline or improvement in the person's physical or medical condition. For instance, when initially placed on the person support apparatus, the person may be feeling good enough to sit upright, but then later the person may have a physical downturn as indicated by one or more measured physiological parameters (e.g., blood pressure, heart rate, etc.), at which time the person support apparatus would reposition itself so that the patient is placed in a safer position (e.g., a reclined position).

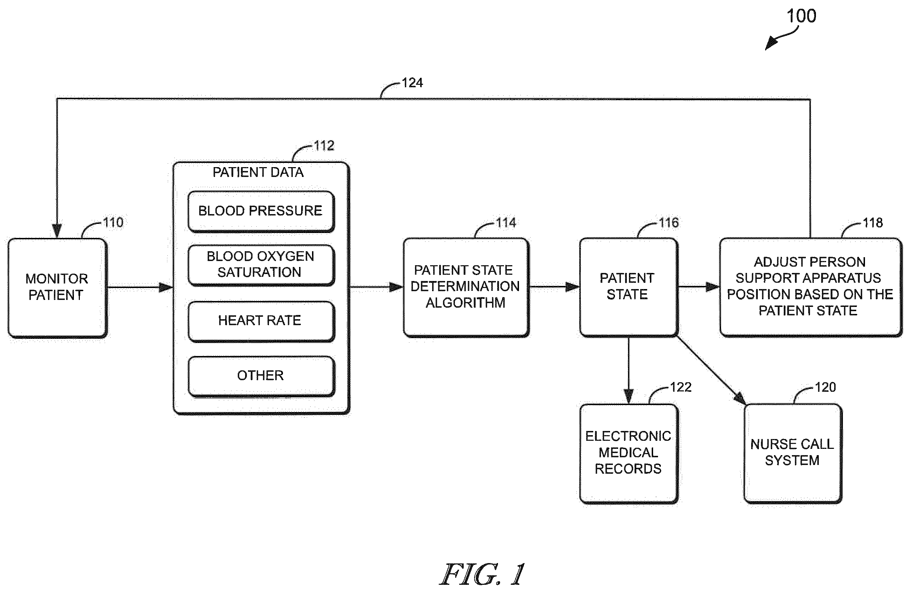

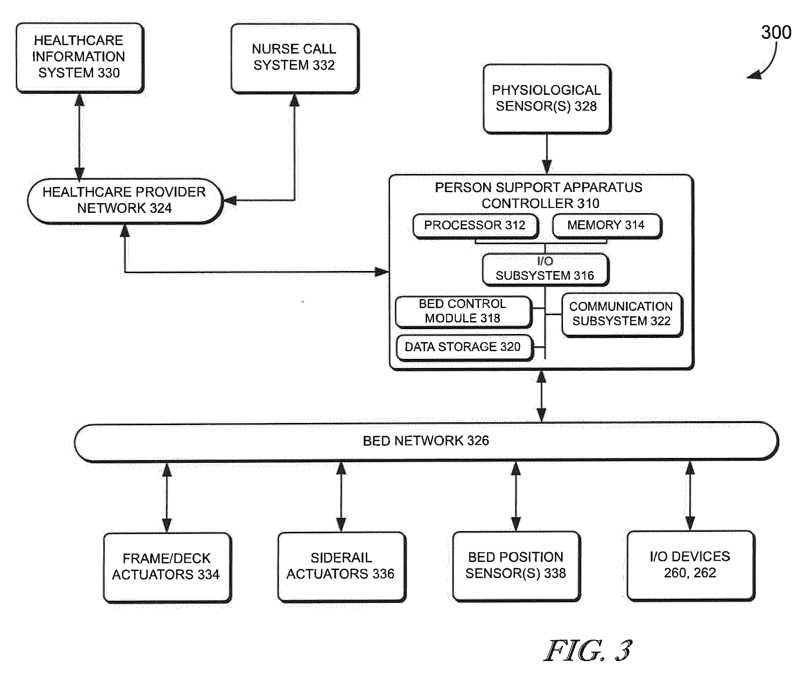

Referring to FIG. 1, a schematic diagram of the functionality of the illustrative person support apparatus control system 100 is shown. At block 110, the system 100 begins monitoring one or more physiological parameters (e.g., vital signs) of a patient, such as a person who has been positioned on the person support apparatus prior or subsequent to a health care event. To do this, the system 100 receives inputs from one or more sensors 328, described in more detail below with reference to FIG. 3. The one or more sensors may be embodied as heart rate monitors, blood oxygen monitors, blood pressure monitors, or other kinds of vital signs monitors. Alternatively or in addition, the sensors 328 may be embodied as more "generic" sensing devices, such as accelerometers or even a camera, coupled with computer programs executing algorithms for extracting and interpreting the sensor data as physiological information. In either case, the system 100 obtains physiological information from the sensor inputs. In some embodiments, the system 100 may perform the patient monitoring of block 110 at discrete time intervals, while in other embodiments, the system 100 may perform the patient monitoring in a continuous fashion for a fixed or variable-length period of time (e.g., while the patient is detected as being situated on the patient support apparatus).

The patient data collected in block 110 is represented by block 112. At block 114, a computer program executing a patient state determination algorithm uses the patient data 112 to determine a "patient state." In some embodiments, the patient state provides an indication of the patient's degree of lucidity, as discussed above. The patient state output by block 114 is represented by block 116. The degree of lucidity is a representation of the degree to which the patient is conscious and alert. The system 100 computes the patient state 116 by, for example, mapping the current values of the patient data 112 to corresponding patient states (e.g., lucidity states), where the mapping between the patient data 112 and the patient states 116 is previously determined based on expert knowledge, research, experimental results, or a combination thereof. For instance, a decreasing heart rate, blood pressure, or blood oxygen saturation, or a combination of these factors, may indicate decreasing lucidity. Conversely, increasing heart rate, blood pressure, and/or blood oxygen saturation levels may indicate that the patient's lucidity is increasing. In some embodiments, the system 100 monitors samples of the patient data 112 over time to detect changes in the patient's state 116, such as abrupt changes or trends that occur over a longer period of time. In some cases, the system 100 may treat rapid changes in one or more of the patient data 112 differently than changes that occur more gradually.

At block 118, the patient state 116 is used to determine the position and configuration of the person support apparatus on which the person being monitored resides or will soon reside (for instance, the system 100 may be used in anticipation of the person support apparatus receiving a particular patient, e.g., after admission or surgery - in other words, in order to pre-configure the person support apparatus for the patient). For example, if the patient data 112 indicates that the patient is highly lucid (i.e., the patient has a high patient lucidity state), the system 100 may cause the person support apparatus to assume a chair position. Conversely, if the system 100 determines that the patient is not lucid (i.e., the patient has a low patient lucidity state), the system 100 may cause the person support apparatus to assume a bed position. Of course, the system 100 may execute logic that results in no changes to the position or configuration of the person support apparatus, in response to the patient's current state. For instance, if there is little change in the patient's state, or if an adjustment is determined to be undesirable for another reason (e.g., other risks or patient preferences), the system 100 may maintain the current position or configuration of the patient support apparatus rather than making an adjustment, in block 118.

At block 120, the patient state is used to determine if the patient state data and/or an alert or other type of notification should be transmitted to another device, e.g., a mobile device of a caregiver, a nurse's station or patient station of a nurse call system, or other type of communication device. For example, if the patient state data indicates that a patient's health is deteriorating rapidly, an alert may be transmitted over a healthcare communication system, such as a nurse call system. At block 122, the patient state and/or the corresponding patient data 112 data may be stored in the patient's electronic medical records. As shown by feedback loop 124, after adjusting the position of the person support apparatus, the system 100 returns to monitoring the patient at block 110.

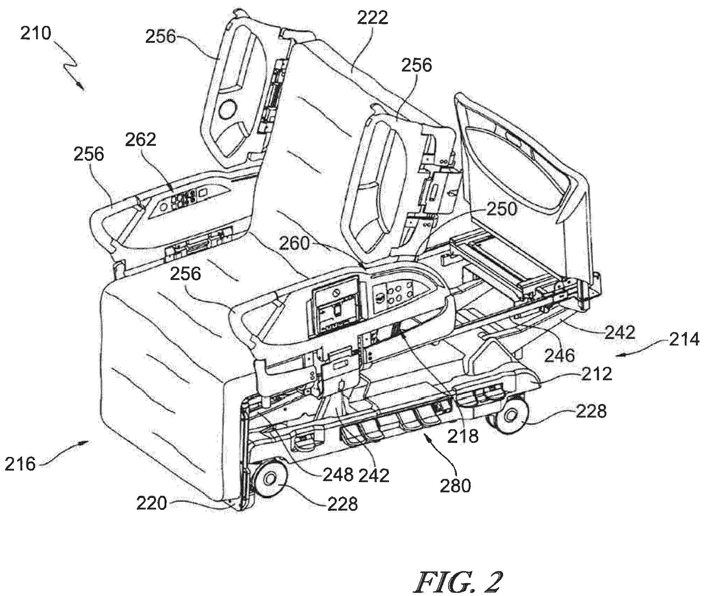

Referring now to FIG. 2, an illustrative embodiment of a person support apparatus 210 is shown. While the illustrative person support apparatus 210 is a type of bed typically used in hospitals and other facilities in which health care is provided, aspects of the present disclosure are applicable to any type person support apparatus that has electronically-controllable features, including but not limited to stretchers, ambulatory stretchers, beds, and other person support structures. For ease of discussion, the term "bed" may be used herein and/or in the drawings to refer to of the aforementioned or other types of person support structures.

The person support apparatus 210 has one or more electronically-controllable functions or features, which may include, but are not limited to: adjusting the position, length, width, or tilt of the person support apparatus, raising, lowering, or pivoting a section of the person support apparatus, raising or lowering a siderail of the person support apparatus, weighing a person positioned on the person support apparatus, inflating, deflating, or adjusting inflation in one or more sections of the mattress, laterally rotating a person positioned on the person support apparatus, providing percussion, vibration, pulsation, or alternating pressure therapy to a person positioned on the person support apparatus, monitoring a person's position or orientation on or relative to the person support apparatus, generating an alert if a person on the person support apparatus changes position or exits the person support apparatus or is in a certain position for too long, weighing a person positioned on the person support apparatus, enabling a person positioned on the person support apparatus to communicate with a caregiver located outside the person's room through an electrical network or telecommunications system, and exchanging data and/or instructions with other devices, equipment, and/or computer systems. Accordingly, the person support apparatus 210 has its own supply of electrical power (e.g. a battery) and/or a connector (not shown) that connects the person support apparatus 210 to a supply of AC electrical power (e.g. a wall outlet).

While the person support apparatus 210 often assumes a flat or horizontal position, FIG. 2 shows the person support apparatus 210 in a chair position. The person support apparatus 210 may assume other positions, as described below. The illustrative person support apparatus 210 includes a base 212, which has a head end 214 and a foot end 216 spaced longitudinally from the head end 214 by the length of the person support apparatus 210. The base 212 is supported by a number of casters 228. The casters 228 each include one or more wheels that movably support the person support apparatus 210 relative to a floor or other surface, in one or more directions. The base 212 and/or one or more of the casters 228 may have an electronically or mechanically-controlled brake and/or steer lock mechanism coupled thereto. A proximity sensor, binary switch, or other suitable type of sensor may be coupled to the caster brake/steer mechanism, and coupled to a person support apparatus controller 310, described below, to enable the controller 310 to monitor the status of the caster brake/steer mechanism. An example of a person support apparatus having a sensor or switch that detects the status of a brake mechanism is disclosed in U.S. Patent No. 6,321,878.

A frame 246 is coupled to and supported by the base 212. A lift mechanism, which includes lift arms 242, is configured to raise, lower, and tilt the frame 246 relative to the base 212. A weigh scale may be coupled to the frame 246. Some examples of person support apparatus with built-in weigh scales and associated displays and user controls are disclosed in U.S. Patent Nos. 4,934,468; 5,715,548; 6,336,235; 7,296,312; and 7,500,280.

The built-in weigh scale may be electronically controlled. For example, the controller 310 may enable a caregiver to weigh a person positioned on the person support apparatus 210 by pressing a button that is electronically connected to the controller 310. The person's weight as determined by the on-board weigh scale may be displayed (e.g. via an LCD display) and stored in memory. If the person support apparatus is not provided with a weigh scale, the controller 310 may enable a caregiver to input the person's weight as determined by other means, for storage in memory and use by the controller 310. Alternatively or in addition, the controller 310 may include computer logic to obtain the person's weight information from an electronic medical records (EMR) database or other stored location. The controller 310 may use the person's weight information to configure pressure settings for a mattress 222 used in connection with the person support apparatus 210, and/or to adjust the articulation of the person support apparatus 210.

A deck 218 is coupled to and supported by the frame 246. The deck 218 is configured to support the mattress 222, which, in turn, may support a person positioned thereon. The deck 218 has a number of sections including, in the illustrated embodiment, an articulating foot section 220 and an articulating head section 250. The deck 218 also includes an articulating torso section 248. In the illustrated embodiment, the torso section 248 includes a separate torso section 248 and seat section (view obstructed). In other embodiments, the torso section 248 may include a single deck section (e.g. a seat/thigh section) rather than two separate deck sections.

The foot section 220 and the head section 250 are pivotable, such that the deck 218 may assume a number of different positions as noted above. In the chair position, the foot section 220 is pivoted downwardly toward the base 212 and the head section 250 is pivoted upwardly away from the frame 246. In the illustrated embodiment, the torso section 248 is also pivotable relative to the frame 246. For example, the torso section 248 may be pivoted upwardly away from the frame 246 to support the patient's knees when the head section 250 is elevated. Other positions that the person support apparatus 210 may assume include a low position, in which the frame 246 is lowered toward the base 212, a Trendelenburg position, a Reverse Trendelenburg position, and any position between the flat position and the chair position. The vertical lift mechanism (e.g., lift arms 242) can raise and lower the frame 246 relative to the base 212. For instance, the frame 246 may be raised to a higher vertical position when the deck 218 is in a flat or "bed" position (e.g., to allow easier maneuvering of the bed 210), and the frame 246 may be lowered when the deck 218 is in the chair position (e.g., to facilitate patient ingress and egress).

While not visible in the view of FIG. 2, the person support apparatus 210 has a number of powered actuators, such as electric linear actuators or hydraulic cylinders, which enable the person support apparatus 210 to assume different positions. One or more actuators are coupled to the frame 246 to enable raising, lowering, and tilting of the frame 246 relative to the base 212. Other actuators are coupled to each of the deck sections 220, 248, 250 to enable pivoting of the deck sections 220, 248, 250 relative to the frame 246. Still other actuators include, actuators coupled to each of one or more siderails 256 to enable motion of the siderails 256 relative to the deck 218 (e.g., raising to a "use" position and lowering to a "storage" position). Examples of such actuators are disclosed in U.S. Patent Nos. 5,715,548; 6,185,767; 6,336,235; 6,694,549; 7,454,805; 6,708,358; 7,325,265; 7,458,119; 7,523,515; 7,610,637; 7,610,638; and 7,784,128.

In general, each of the actuators is coupled to a power plant (e.g. a motor) and has an extending/retracting arm or linkage. One end of the arm or linkage is coupled to the power plant and the other end is coupled to the frame 246 or the relevant deck section 220, 248, 250. The power plant drives the arm or linkage in one direction to provide movement of the frame 246 or deck section 220, 248, 250 in one direction (e.g. raising or pivoting upwardly), and drives the arm or linkage in the opposite direction to provide movement of the frame 246 or deck section 220, 248, 250 in the other direction (e.g. lowering or pivoting downwardly). The power plant is responsive to control signals issued by the controller 310. When movement of a person support apparatus section is requested, the controller 310 determines the duration of the requested movement (i.e. how far the associated arm or linkage is to be extended or retracted, as the case may be) and the speed at which the requested movement is to be accomplished (i.e. how slowly or quickly the associated arm or linkage is to be extended or retracted), and sends a corresponding control signal or signals to the power plant.

The person support apparatus 210 may include one or more sensors that are coupled to the actuators to monitor the speed or progress of movement or articulation of a person support apparatus section. For example, a bed-not-down sensor may be coupled to the foot section 220 of the deck 218 and/or to the lift mechanism 242, to alert a caregiver if the person support apparatus 210 is not in a position that is suitable for egress, or for other reasons. In response to output of a bed-not-down sensor, the controller 310 may issue a visual and/or audible signal and/or communication signal indicating that the person support apparatus or a section thereof is not in its low or 'down' position.

The person support apparatus 210 may be equipped with additional sensors that are configured to detect other conditions of the person support apparatus. For example, the person support apparatus 210 may have position sensors (such as force sensors) that detect force applied to the bed at different locations on the bed, e.g., for patient position monitoring. In these embodiments, the controller 310 includes executable instructions that determine, based on the output of the force sensor or sensors, the position of a patient relative to the person support apparatus (e.g. the patient has exited the person support apparatus, is on the edge of the person support apparatus, or is sitting up in person support apparatus). The controller 310 may then issue a visual and/or audible signal and/or communication signal relating to the patient's position. Some examples of a person support apparatus having patient monitoring features are disclosed in U.S. Patent Nos. 6,067,019; 6,133,837; 6,208,250; 6,791,460; and 7,464,605.

The person support apparatus 210 may be equipped with one or more physiological sensors that are configured to detect the physiological responses of a person positioned in the person support apparatus 210. For example, some person support apparatus 210 may include a blood pressure sensor and/or a heart rate monitor to measure the blood pressure and the heart rate of the person positioned in the person support apparatus 210. As described above, the controller 310 includes executable instructions that determine, based on the outputs from the one or more physiological sensors, a patient state (e.g., a physical, mental, or physical and mental, state). In some embodiments, the controller 310 issues alerts or bed positioning commands based on the patient state. The one or more physiological sensors may be configured to communicate with the controller 310 through any wired or wireless communication link. For example, Ethernet, Wi-Fi, Bluetooth, Near Field Communications, or other types of communication networks. In response to a patient lucidity state, the controller 310 may issue a visual and/or audible signal and/or communication signal through user interface devices 260, 262 indicating the patient state.

The controller 310 may enable a caregiver to turn patient monitoring features on or off for a particular patient, or to configure a patient monitoring feature differently for different patients or differently for different patient conditions. For example, the caregiver may configure the patient state monitoring feature to operate in a recovery mode or in an observation mode. In the recovery mode, the patient state monitoring feature monitors the patient for indications that the patient's condition is improving, e.g., that the patient is returning to a lucid state (e.g., after some type of healthcare procedure in which the patient may be coming out of anesthesia). In the observation mode, the patient state monitoring feature monitors the patient for any change in that patient's state that may be cause for concern (e.g., a trend of declining lucidity or a rapid decrease in lucidity, which could indicate a fainting episode or other medical event). In some cases, patient state monitoring may be triggered by a change in the patient's position relative to the person support apparatus. For instance, the caregiver may configure the patient monitoring feature to only send an alert if the patient's state has changed and the patient has exited the person support apparatus, while for another patient, the caregiver may configure the patient monitoring feature to send an alert if the patient's state has changed and the patient is detected as sitting on the edge of the person support apparatus.

The person support apparatus 210 may be equipped with angle or orientation sensors, such as ball switches, potentiometers, inclinometers, accelerometers, etc., which detect changes in the orientation of the person support apparatus or one section of the person support apparatus relative to another section of the person support apparatus. For example, an angle sensor may be used to determine the angle of the head section 250 or the foot section 220 of the person support apparatus relative to the frame 246 or to the horizontal. The controller 310 includes executable instructions that determine, based on the output of the orientation sensor or sensors, the orientation of the person support apparatus or section(s) thereof. The controller 310 may then issue a visual and/or audible signal and/or communication signal relating to the person support apparatus' orientation. For example, the controller 310 may alert a caregiver if the angle of the head section 250 is less than 30 degrees above horizontal. An example of a person support apparatus that has a head angle alarm feature is disclosed in U.S. Patent No. 7,487,562.

The person support apparatus 210 may be equipped with pressure sensors, such as transducers, strain gauges, capacitive, optical or piezoelectric sensors, or the like, which detect changes in pressure applied to different sections of the mattress 222 or pressure inside of the person support apparatus' mattress (if the person support apparatus' mattress has air bladders). The controller 310 includes computer-executable instructions that determine, based on the output of a pressure sensor or sensors, the pressures within air bladders or zones of air bladders of the mattress 222. The controller 310 may then determine that a bed condition has occurred based on the pressure sensor output, such as a bottoming out condition or a max-inflate condition. The controller 310 may alternatively or in addition issue control signals to inflate or deflate certain air bladders based on the output of the pressure sensors, as may be the case when the bed is operating in a pressure relief mode or a therapy mode. The controller 310 may issue a visual and/or audible signal, and/or a communication signal relating to the mattress condition or status. Some examples of person support apparatus having sensors responsive to mattress conditions are disclosed in U.S. Patent Nos. 6,505,368; 7,260,860; 7,330,127; 7,469,436; and 7,617,555.

The illustrative person support apparatus 210 includes a number of siderails 256, a pair of opposing endboards (e.g. a headboard and a footboard, not shown). A proximity sensor, switch, or other suitable device may be coupled to the siderails and to the controller 310 to detect when the siderails are up or down. The controller 310 may then issue a visual and/or audible signal and/or communication signal relating to the status of the siderails 56, 58. For example, the controller 310 may alert a caregiver if one or more of the siderails 56, 58 are down. An example of a person support apparatus having a siderail down sensor is disclosed in U.S. Patent No. 6,021,533.

The electronically-controllable features and functions of the person support apparatus 210 may be activated, configured, and deactivated by user inputs that are translated into electrical signals and forwarded to the controller 310 by input devices or input-output devices such as foot pedals, buttons, switches, dials, slides, and the like, as well as graphical user interface modules and/or touchscreens. Portions of the controller 310 may be embodied in the user interface device 260, 262.

The illustrative person support apparatus 210 has a number of foot pedals 280. The foot pedals 280 are coupled to and supported by the base 212. The foot pedals 280 are in electrical communication with the controller 310 and may be used by a caregiver to change the position of the person support apparatus 210, or to control the casters (e.g. activate or deactivate a brake or steer lock mechanism), or to activate or deactivate some other feature of the person support apparatus 210. Stepping on a foot pedal issues a control signal to the controller 310. Some examples of a person support apparatus with foot-operated controls are disclosed in U.S. Patent Nos. 6.691,346; 6,978,500; and 7,171,708.

The person support apparatus 210 also has user interface devices 260, 262, which are configured to permit caregivers and patients, as the case may be, to activate and deactivate certain electronically-controllable features of the person support apparatus 210, to view information (such as patient lucidity information) displayed on a graphical user interface, and perform other functions.

The user interface device 260 receives and processes electrical input (e.g. voltage) from one or more controls mounted thereto, which enable a user (e.g., a caregiver) to configure, activate and/or deactivate certain of the electronically-controllable person support apparatus functions. For example, some person support apparatus permit the caregiver to raise and lower the person support apparatus or change the position of certain sections thereof, change the length or width of the person support apparatus, or to achieve a chair, CPR, Trendelenburg, or reverse Trendelenburg position, or to activate certain mattress therapies (such as lateral rotation, percussion, or vibration), by physically contacting the selected control. In some embodiments, the user interface device 260 includes one or more buttons that, for example, enable the caregiver to place the person support apparatus 210 into a chair position in which the head section 250 is elevated and the foot section 220 is rotated downwardly toward the floor. In some embodiments, the user interface device 260 a graphical touchscreen user interface that has a number of menus and controls that allow a user to activate, deactivate, or configure features of the person support apparatus 210.

In some embodiments, the user can optionally enable the patient state monitoring features of the person support apparatus 210. For example, after enabling the patient state monitoring features, the caregiver may exit the patient state monitoring features by adjusting the position of the person support apparatus 210.

The user interface device 260 includes circuitry configured to convey voltage generated by its controls to the controller 310. In the illustrated embodiment, the user interface device 260 is mounted to the outwardly facing side of at least one of the siderails 256 of the person support apparatus 210 (i.e., facing away from the mattress), but the user interface device 260 may be placed in any suitable location that is accessible to the appropriate user (e.g., a caregiver). For example, some user interface controls may be provided on a wall-mounted device or a remote device.

The user interface device 262 receives and processes electrical input (e.g. voltage) from number of manually operable controls (such as membrane switches, keys, dials, levers, or the like) coupled to the user interface device 262, which enable a user (e.g., a patient) to activate and deactivate certain person support apparatus functions when the user is positioned on the person support apparatus 210. For example, the person support apparatus may permit the user to raise and lower the person support apparatus or change the position of certain sections thereof by touching these controls.

The user interface device 262 includes circuitry to convey voltage generated by the manually operable controls to the controller 310. In the illustrated embodiment, the user interface device 262 is mounted to the inwardly facing side of at least one of the siderails 256 of the person support apparatus 210 (i.e., facing toward the mattress), but the user interface device 262 may be placed in any suitable location that is accessible to a person using the person support apparatus 210. For example, some patient controls may be provided on a pendant controller or a remote device.

Referring now to FIG. 3, an embodiment 300 of the person support apparatus control system 100 is shown. The illustrative person support apparatus control system 300 includes the user interface devices 260, 262, the person support apparatus controller 310, described above, a healthcare provider network 324, physiological sensor(s) 328, a healthcare information system 330, a nurse call system 332, frame/deck actuators 334, siderail actuators 336, and bed position sensor(s) 338. The components of the system 300 include computer hardware, software, firmware, or a combination thereof, configured to perform the features and functions described herein.

The illustrative person support apparatus controller 310 includes hardware, firmware, and/or software components that are capable of performing the functions disclosed herein, including the functions of a bed control module 318. In some embodiments, the controller 310 or portions thereof may be embodied as electrical circuitry, e.g., hardware built-in to the person support apparatus 210. The illustrative controller 310 includes at least one processor 312 (e.g. a controller, microprocessor, microcontroller, digital signal processor, etc.), memory 314, and an input/output (I/O) subsystem 316. Portions of the person support apparatus controller 310 may be built-in to the person support apparatus 210 or embodied in any type of computing device capable of performing the functions described herein, such as any type of general purpose computing device, specialized computing device, consumer-oriented computing device, mobile electronic device (e.g., a tablet computer, smart phone, body-mounted device or wearable device, etc.), a server, an enterprise computer system, a network of computers, a combination of computers and other electronic devices, or other electronic devices. Although not specifically shown, it should be understood that the I/O subsystem 316 typically includes, among other things, an I/O controller, a memory controller, and one or more I/O ports. The processor 312 and the I/O subsystem 316 are communicatively coupled to the memory 314. The memory 314 may be embodied as any type of suitable computer memory device (e.g., volatile memory such as various forms of random access memory).

The I/O subsystem 316 is communicatively coupled to a number of hardware, firmware, and/or software components, including the bed control module 318, a data storage device 320, and a communication subsystem 322. The data storage device 320 may include one or more persistent data storage devices (e.g., flash memory, memory cards, memory sticks, and/or others). Data used by the controller 310 (e.g., physiological sensor data) resides at least temporarily in the data storage device 320 and/or other data storage devices of the system 300 (e.g., data storage devices that are "in the cloud" or otherwise connected to the controller 310). Portions of the bed control module 318 may reside at least temporarily in the data storage device 320 and/or other data storage devices that are part of the system 300. Portions of the physiological data or the bed position data may be copied to the memory 314 during operation of the controller 310, for faster processing or for other reasons.

The communication subsystem 322 may communicatively couple the controller 310 to other computing devices and/or systems by, for example, one or more networks 324, 326. Portions of the network(s) 324, 326 may be embodied as any suitable type of network capable of performing the functions described herein, including any suitable wired, wireless, or optical communication technology. Accordingly, the communication subsystem 322 may include one or more optical, wired and/or wireless network interface subsystems, cards, adapters, or other devices, as may be needed pursuant to the specifications and/or design of the particular controller 310.

The illustrative communication subsystem 322 communicates outputs of the bed control module 318 to the healthcare information system 330 and/or the nurse call system 332, via a healthcare provider network 324. For example, the patient state may be supplied to the healthcare information system 330 or the nurse call system 332. Additionally, the illustrative communication subsystem 322 communicates outputs of the bed control module 318 (e.g., commands or control signals) to the frame/deck actuators 334, the siderail actuators 336, and the I/O devices 260, 262, via a bed network 326. For example, the patient state may be used to determine bed positioning or adjustment commands to be sent to one or more electromechanical components of the person support apparatus 210. The bed control module 318 is configured to determine a patient state and generate alerts and bed position commands based on the patient state, as described herein.

The controller 310 may be connected to the healthcare provider network 324, which connects the person support apparatus 210 to a hospital or other facility in or in connection with which the person support apparatus 210 is used, via a bidirectional signal path, in order to send and/or receive data and/or instructions to/from the healthcare information system 330 or the nurse call system 332. The healthcare information system 330 may include one or more networked systems, such as an admission, discharge, and transfer (ADT) system and an electronic medical records (EMR) system. An example of a system in which a person support apparatus network communicates with an ADT system is disclosed in U.S. Patent Application Serial Nos. 12/708,891, filed February 19, 2010, and 12/711,912, filed February 24, 2010. It will be understood that some of these processes and systems, or portions of them, may not be performed by or physically located at the facility in which the person support apparatus is used. For example, data storage and/or processing, or portions thereof, may be performed by other entities or at other locations.

The illustrative nurse call system 332 is also connected to the controller 310 through the healthcare provider network 324. The nurse call system 332 may be any system designed to aid healthcare professionals to provide care to a patient. For example, a nurse call system may alert healthcare professionals when a patient is experiencing an unsafe condition. In an illustrative embodiment, the nurse call system 332 is configured to alert nurses or other healthcare facility staff about changes in the patient's state, as determined by the controller 310.

As discussed above, one or more sensor(s) 328 are in communication with to the controller 310, and the sensor(s) 328 are configured to detect or measure the physiological responses of the person positioned in the person support apparatus 210. In some embodiments, the physiological sensors 328 may include a blood pressure sensor, a blood oxygen saturation sensor, a heart rate monitor, or an electroencephalography (EEG) sensor to measure the ionic current flows within the neurons of the brain. Other types of physiological sensor(s) are also included in this disclosure. The physiological sensors 328 are in communication with the controller 310 through either wired or wireless (including optical) communication technology. The physiological sensors 328 provide physiological data that the controller 310 uses to determine the patient state as described herein.

The controller 310 communicates with the frame/deck actuators and the siderail actuators 336 through the bed network 326. The frame/deck actuators 334 are coupled to the frame 246 and the deck 218, and are configured to raise, lower, or tilt the frame 246 and deck 218 relative to the base 212. In an illustrative embodiment, the frame/deck actuators 334 can cause the head section 250 to pivot relative to the torso section 248, and/or cause the foot section 220 to pivot relative to the torso section 248, and/or cause the torso section to pivot relative to the frame 246 or the base 212. The frame/deck actuators can also cause the deck 218 to vertically raise and lower relative to the base 212. For example, the frame/deck actuators 334 can lower the person support apparatus 210 when the person support apparatus 210 functions as a chair because the seat of most chairs is in the range of about seventeen inches above the ground. Whereas, the frame/deck actuators 334 can raise the person support apparatus 210 when the person support apparatus 210 functions as a bed because most hospital beds/stretchers are in the range of approximately forty-five inches above the ground.

The controller 310 also communicates with and controls the siderail actuators 336. As discussed above, the siderails 256 are coupled to the frame 246 or deck 218 and are configured to be positioned by the siderail actuators 336. In some embodiments, the siderails 256 can be positioned in a number of different positions between a fully raised position and fully down position. In an illustrative embodiment, when the person support apparatus 210 is in a chair position, the siderails 256 can be in the fully down position; and when the person support apparatus 210 is in a bed position, the siderails 256 are in the fully raised position. Illustratively, the controller 310 positions the person support apparatus 210 in a bed position when the patient using the person support apparatus 210 is not very lucid, or has a low patient lucidity state. When the patient has a low patient lucidity state, the siderails 256 are fully raised to prevent the patient from falling off of the person support apparatus 210. In some embodiments, opposing siderails 256 are coupled to the foot deck section 220, the torso deck section 248, and the head deck section 250.

The controller 310 is in communication with one or more bed position sensor(s) 338 through bed network 326. The bed position sensor(s) 338 are configured to detect the position of the person support apparatus 210, so that the controller 310 can effectively reposition the person support apparatus after the patient state has changed. Many states of the bed can be detected by the bed position sensor(s) 338, such as, for example, the angle between the torso section 248 and the head section 250, the angle between foot section 220 and the torso section 248, torso section angle, the height of the torso section 248 above the base 212, the height of the head section 250 above the base 212, or various other settings related to the mattress or the person support apparatus 210.

The controller 310 is also coupled to the user interface devices 260, 262 to provide both the caregiver and the user of the person support apparatus 210 control over the various functionalities of the person support apparatus 210. The user interface devices 260, 262 may include one or more user input devices (e.g., a microphone, a touchscreen, keyboard, virtual keypad, etc.) and one or more output devices (e.g., audio speakers, LEDs, additional displays, etc.). The display may be embodied as any suitable type of digital display device, such as a liquid crystal display (LCD), and may include a touchscreen.

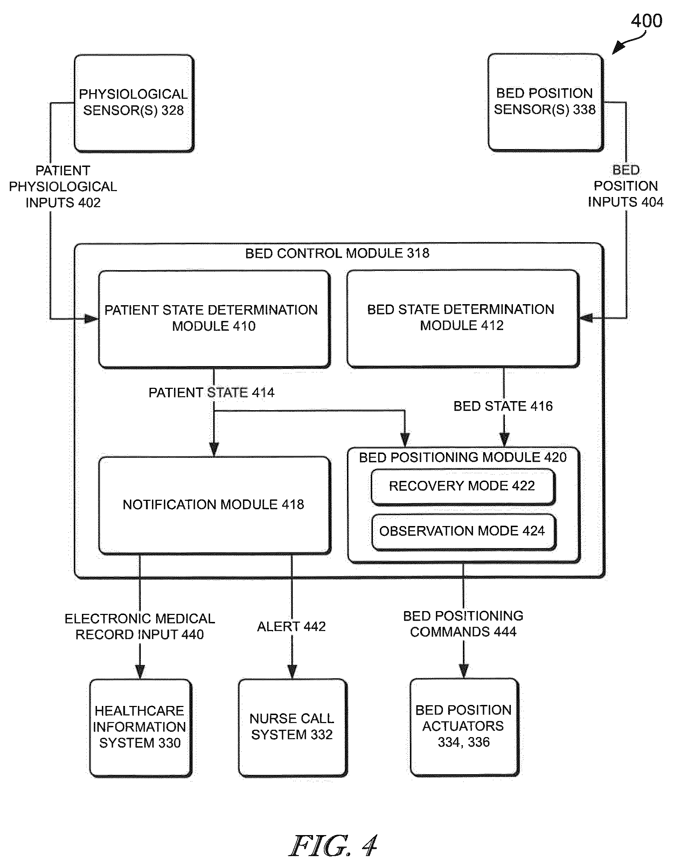

Referring now to FIG. 4, a simplified schematic diagram shows components of the system 300 in an operational computing environment 400 (e.g., interacting at runtime). The components of the person support apparatus control system 300 shown in FIG. 4 may be embodied as computerized programs, routines, logic, data, and/or instructions executed or processed by the controller 310. As shown in FIG. 4, the bed control module 318 obtains or receives patient physiological inputs 402 from the physiological sensor(s) 328 (discussed above) and obtains or receives bed position inputs 404 from the bed position sensor(s) 338 (also discussed above). The illustrative bed control module 318 uses both physiological inputs 402 and bed position inputs 404 to determine various outputs, such as, electronic medical record inputs 440, nurse alerts 442, and bed positioning commands 444. To produce these outputs, the bed control module 318 includes a patient state determination module 410, a bed state determination module 412, a notification module 418, and a bed positioning module 420.

The patient state determination module 410 receives and analyzes the physiological inputs 402 from the physiological sensor(s) 328 and determines a patient state 414. The patient state 414 is determined by evaluating the physiological responses of the patient that are measured by the physiological sensor(s) 328. In some embodiments, the types of physiological responses that can be measured include blood pressure, blood oxygen saturation, and heart rate, just to name a few. Using these physiological inputs 402, the patient state determination module 410 determines the degree to which the patient is mentally or physically well, i.e., lucid, e.g., in control of his or her mental faculties. Lucidity as used herein may refer to, among other things, a way to quantify how much control a patient has over his or her own body functions. For example, a patient that is fully lucid will have complete control over his or her faculties, and vice versa. The patient state 414 can be illustratively defined as a number of discrete states with a person support apparatus position corresponding to each discrete state. For example, the patient state 414 can include a recovering state, an under stress state, a requiring-attention state, an unconscious state, a sleeping state, or a completely lucid state. In the example just recited, if the patient is experiencing the unconscious state just mentioned, the corresponding position of the person support apparatus 210 would be a reclined, bed or bed-like position. Conversely, in the example just recited, if a patient is experiencing the completely lucid state just mentioned, the corresponding position of the person support apparatus 210 would be an upright, chair, or chair-like position. In some embodiments, the patient state determination module 410 can discern between a sleeping patient and a patient who is losing consciousness. In some embodiments, the patient state determination module 410 can also compare the current patient state to previously calculated patient states that are stored by the controller 310. By comparing former patient states to the current patient state 414, the patient state determination module 410 can determine the rate of change of the patient state 414 over time.

In an illustrative embodiment, the patient state 414 is sent to the notification module 418. The notification module 418 determines whether the patient state needs to be reported out to other systems. For example, if the patient state 414 indicates that the patient is rapidly changing from a lucid state to a non-lucid state, then the notification module 418 can generate an alert 442 to be transmitted to the nurse call system 332. In the situations where a patient is in need of immediate care, the nurse call system 332 can broadcast the alert 442 to many healthcare professionals at once, alerting the healthcare professionals of the needs of the particular patient. In some embodiments, the notification module 418 transmits electronic medical record inputs 440, which includes the patient state 414 and the physiological inputs 402, to a healthcare information system 330 to be included in the electronic medical records of the patient.

The bed state determination module 412 receives the bed position inputs 404 from the bed position sensor(s) 338 and determines a bed state 416. The bed state 416 comprises the current position of the person support apparatus 210. The patient state 414 and the bed state 416 are both sent to the bed positioning module 420. The bed positioning module 420 determines a new bed position for the person support apparatus 210 based on the patient state 414. The bed positioning module 420 also generates bed positioning commands 444 based on the bed state 416 and the desired new bed position.

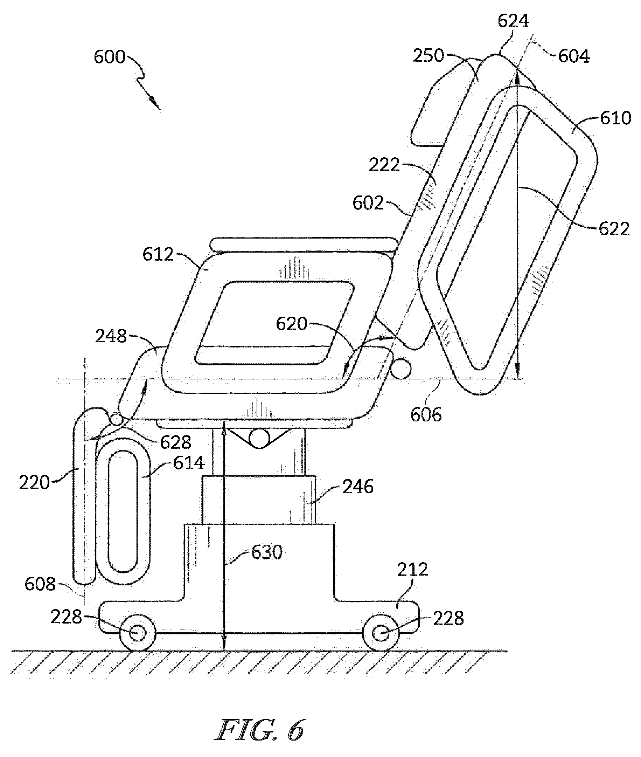

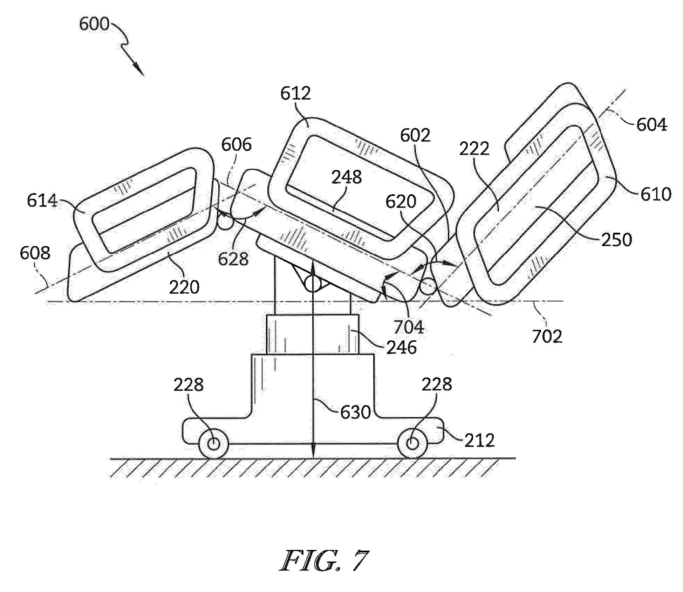

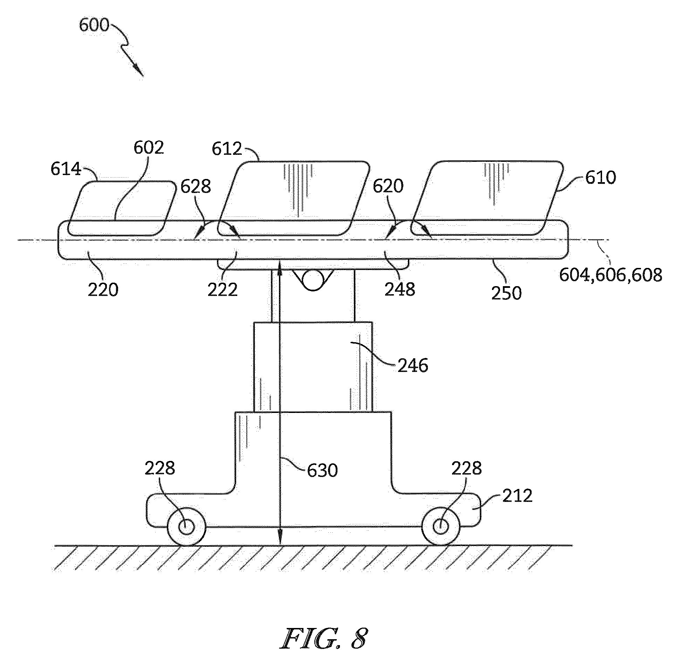

The bed positioning module 420 includes a recovery mode 422 and an observation mode 424. In some embodiments, the recovery mode 422 and the observation mode 424 can have different bed positions for the same patient state 414. The recovery mode 422 is used when a patient is recovering from some type of surgery or outpatient care, such as, for example, when a patient is coming out of anesthesia. When in recovery mode 422, the patient starts in a not lucid state and should be becoming more lucid over time. In some embodiments, when in recovery mode 422, the patient state determination module 410 is expecting the physiological inputs 402 to show signs of more lucidity. For example, the patient's blood pressure and heart rate should be increasing. In recovery mode 422, the person support apparatus 210 is initially configured to be in a bed position with the siderails 256 fully raised to, for example, prevent the non-lucid patient from falling, as shown in FIG. 8. When in recovery mode 422, the person support apparatus 210 can be gradually or progressively repositioned from the bed position to the chair position. As the patient state 414 indicates that the patient is becoming more lucid, the person support apparatus 210 is gradually repositioned by raising the head section 250 and lowering the foot section 220. An illustrative example is shown in FIG. 7, described below. As a patient becomes completely lucid, as indicated by the patient state 414, the person support apparatus 210 may be adjusted to a chair position, as shown in FIG. 6, described below. The rate at which the person support apparatus 210 is repositioned corresponds to the rate at which the patient's physical and/or mental state changes. The correlations between rate of change of the patient support position and rate of change of patient state can be implemented in, for example, a mapping table or database, which may be developed based on, e.g., expert knowledge, experimental test results, or by other suitable methods.

The observation mode 424 may be used when a patient's condition is unknown and the patient is to be monitored for an uncertain amount of time. In some embodiments, in the observation mode 424, the person support apparatus 210 is initially configured to be in a chair position. The person support apparatus 210 can react to a declining patient state 414 by reclining and tilting the person support apparatus 210 into a safer position. For example, a safer person support apparatus position can be achieved by lowering the head section 250, raising the foot section 220, and raising the siderails 256. In some embodiments, both the recovery mode 422 and the observation mode 424 of the person support apparatus positioning system 300 can be exited by either the caregiver or the patient using the I/O devices 260, 262. For example, if the patient's state deteriorates, a healthcare provider can use the I/O devices 260, 262 to transform the person support apparatus into a bed position for added safety and continued monitoring. In some embodiments, observation mode 424 can be initiated with the person support apparatus 210 in any position. For example, if a person is placed under doctor observation while in a semi-lucid state, the healthcare provider might configure the person support apparatus 210 into an intermediate bed position between a chair position and a bed position) before beginning the observation mode 424. In this situation, the bed positioning module 420 would adjust the positioning of the person support apparatus 210 starting from this initial intermediate bed position.

The person support apparatus control system 300 can provide a number of benefits to both patients and caregivers. For recovering patients, a gradually elevating the head is often better than moving immediately from a flat position to a standing or seated position, and can reduce post-operative falls. Another advantage is that transferring a patient from a stretcher or bed to a chair does not require as much assistance from the caregivers. Another advantage is that patient dignity can be increased, while still maintaining the patient in a safe position.

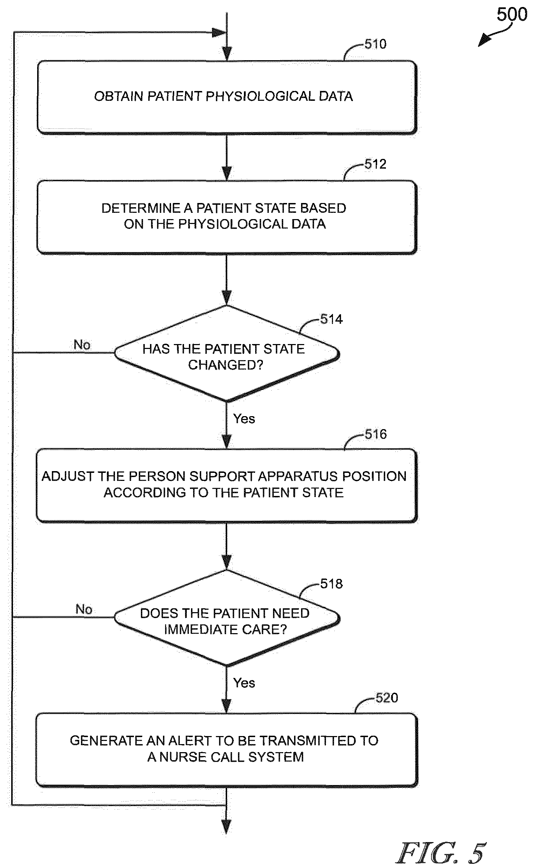

Referring now to FIG. 5, an illustrative method 500 for adjusting the position of a person support apparatus 210 based on a patient state is shown. Aspects of the method 500 may be embodied as computerized programs, routines, logic and/or instructions executed by the person support apparatus positioning system 300, for example the controller 310. At block 510, the system 300 obtains physiological inputs 402 from the physiological sensor(s) 328 connected to the patient positioned in the person support apparatus 210. Using the physiological data collected about the patient, at block 512, a patient state is determined. The patient state measures how conscious or alert a patient is.

At block 514, the system 300 determines if the patient's physical and/or mental state has changed. If the patient state has not changed, then the system 300 continues to obtain physiological data, at block 510, and determine patient states, at block 512. If the patient state has changed, then, at block 516, the system 300 adjusts the position of the person support apparatus 210 according to the new patient state. For example, if the patient state indicates that a patient has become less lucid, then the person support apparatus might be repositioned to lower the head position, raise the feet position, and raise the siderails.

At block 518, the system 300 determines if a patient needs immediate care. Not all changes to the patient state require the immediate attention of a caregiver. For example, no immediate care is needed if the system 300 determines that a patient has fallen asleep, but immediate care might be necessary if the person is become less lucid for other reasons (e.g., a heart attack). If no immediate care is required, then system 300 returns to block 510 to again monitor the patient's physiological responses. If immediate care is required, then, at block 520, the system 300 generates an alert to be transmitted to a nurse call system. In some embodiments, the alert is configured to cause any available healthcare provider to come to the assistance of the patient. After block 520, the method 500 returns to block 510 to continue monitoring the patient's physiological responses.

Referring to FIGS. 6-8, another illustrative embodiment of a person support apparatus 600 is shown. The person support apparatus 600 is, for example, a stretcher/chair device. The person support apparatus 600 may include any of the features or functions of the person support apparatus 210, described above, however. FIG. 6 shows the person support apparatus 600 in a chair position, FIG. 7 shows the person support apparatus 600 in an intermediate position, and FIG. 8 shows the person support apparatus 600 in a bed position. For ease of discussion, only three different person support apparatus 210 positions are discussed herein; however, it should be appreciated that many other different positions exist, including positions intermediate4 each of these three positions.

The person support apparatus 600, as configured in a chair position, and corresponding to a position associated with a high patient lucidity state, is shown in FIG. 6. The illustrative person support apparatus 600 includes a base 212, a frame 246, and a deck 218. The base 212 is supported by one or more casters 228. The deck 218 includes a head section 250, a torso section 248, and a foot section 220. A mattress 222 is configured to rest on top of the deck 218, and defines a mattress upper surface 602. Each of the deck sections 220, 248, 250 defines an imaginary plane running the length of the deck section. The head section 250 defines a head section plane 604, the torso section 248 defines a torso section plane 606, and the foot section defines a foot section plane 608.

Siderails are coupled to each of the sections 220, 248, 250. The siderails include a head siderail 610 coupled to the head deck section 250, a torso siderail 612 coupled to the torso deck section 248, and a foot siderail 614 is coupled to the foot section 220. In the illustrative example, the head siderail 610 and the foot siderail 614 are in an undeployed position, meaning that the siderails do not extend above the upper surface 602 of the mattress 222. However, in the illustrative embodiment, the torso siderail 612 is in a fully deployed position, and acts as an armrest for the patient positioned in the person support apparatus 600.

The head section 250 and the torso section 248 cooperate to define a head-torso angle 620 and a head-torso height 622. The head-torso angle 620 is defined as the angle between the head section plane 604 and the torso section plane 606. In some embodiments, the head-torso angle is between 90 and 180 degrees. Actuators (not shown) are used to pivot the head section 250 in relation to the torso section 248, and thereby change the head-torso angle 620. In some embodiments, changing the head-torso angle 620, in general, also adjusts the head-torso height 622. The head-torso height 622 is defined as the distance between the torso section 248 and a head end 624 of the head section 250.

The torso section 248 and the foot section 220 cooperate to define a foot-torso angle 626. The foot-torso angle 626 is defined as the angle between the foot section plane 608 and the torso section plane 606. The torso section 248, the frame 246, and the base 212 cooperate to define a torso section height 630. The torso section height is defined as the distance between the base 212 and the torso section 248. The torso section height 630 is adjustable according to the desired distance between the deck 218 of the person support apparatus 210 and the ground. For example, the torso section height 630 may be in the range of approximately between sixteen and twenty-two inches when the person support apparatus 210 is in the chair position. In another example, the torso section height can be in the range of about forty inches or more, when the person support apparatus 210 is in the bed position.

The person support apparatus 600, as configured in an intermediate position (i.e., not fully in a chair position, and not fully in a bed position) is shown in FIG. 7. While a particular intermediate position is shown in FIG. 7, it should be appreciated that a number of different intermediate positions are included in this disclosure. For example, an intermediate position could include the person support apparatus 210 being mostly in a bed position, but with the head section 250 pivoted such that the head-torso angle 620 is less than one hundred eighty degrees. In general, an intermediate state of the person support apparatus 210 corresponds to a patient state that indicates that the patient is semi-lucid. Many of the features and parts of the person support apparatus 210 are identical to the features and parts described in FIG. 6, and as such a description of those features and parts are not repeated here in detail. Features having similar names or similar reference numbers may be embodied similarly.

As shown in FIG. 7, the person support apparatus 600 is in a semi-reclined position, which corresponds to a patient state indicating that the patient is semi-lucid. FIG. 7 shows an imaginary horizontal plane 702, which is parallel to the ground that the base 212 rests on. The torso section 248 of the deck 218 is tilted by actuators (not shown) in the frame 246 and defines a torso section angle 704. The torso section angle 704 is defined as the angle between the torso section plane 606 and the imaginary horizontal plane 702. While the torso section angle 704 affects the overall position of the patient, the torso section angle 704 does not affect the head-torso angle 620 or the foot-torso angle 626. All of these angles 620, 626, 704 can be independently set by the controller 310 using frame/deck actuators 334.

The illustrative embodiment of the person support apparatus 600 shown in FIG. 7 has the head siderail 610 and the foot siderail 614 in a partially-deployed position. The partially deployed siderails 610, 614 provide greater safety to the semi-lucid patient because a barrier is placed between the patient and falling from the bed, but the barrier does not completely obstruct the patient's view, allowing for increased patient dignity.

The person support apparatus 600, as configured in a bed position is shown in FIG. 8. In general, the bed position of the person support apparatus 600 in FIG. 8 corresponds to a patient state that indicates that the patient is not lucid. The illustrative embodiment of the person support apparatus 600, as shown in FIG. 8, has all deck sections 220, 248, 250 aligned to form a bed surface that is parallel to the ground. The siderails 610, 612, 614 are fully deployed to prevent patient from egressing from the person support apparatus 210. The torso section height 630 is raised when the person support apparatus 600 is in the bed position of FIG. 8, to allow for easier maneuvering of the person support apparatus 600, or for other reasons.

In the foregoing description, numerous specific details, examples, and scenarios are set forth in order to provide a more thorough understanding of the present disclosure. It will be appreciated, however, that embodiments of the disclosure may be practiced without such specific details. Further, such examples and scenarios are provided for illustration, and are not intended to limit the disclosure in any way. Those of ordinary skill in the art, with the included descriptions, should be able to implement appropriate functionality without undue experimentation.

References in the specification to "an embodiment," etc., indicate that the embodiment described may include a particular feature, structure, or characteristic, but every embodiment may not necessarily include the particular feature, structure, or characteristic. Such phrases are not necessarily referring to the same embodiment. Further, when a particular feature, structure, or characteristic is described in connection with an embodiment, it is believed to be within the knowledge of one skilled in the art to effect such feature, structure, or characteristic in connection with other embodiments whether or not explicitly indicated.

Embodiments in accordance with the disclosure may be implemented in hardware, firmware, software, or any combination thereof. Embodiments may also be implemented as instructions stored using one or more machine-readable media, which may be read and executed by one or more processors. A machine-readable medium may include any mechanism for storing or transmitting information in a form readable by a machine. For example, a machine-readable medium may include any suitable form of volatile or non-volatile memory. Modules, data structures, and the like defined herein are defined as such for ease of discussion, and are not intended to imply that any specific implementation details are required. For example, any of the described modules and/or data structures may be combined or divided into sub-modules, sub-processes or other units of computer code or data as may be required by a particular design or implementation of the system 100. In the drawings, specific arrangements or orderings of schematic elements may be shown for ease of description. However, the specific ordering or arrangement of such elements is not meant to imply that a particular order or sequence of processing, or separation of processes, is required in all embodiments. In general, schematic elements used to represent instruction blocks or modules may be implemented using any suitable form of machine-readable instruction, and each such instruction may be implemented using any suitable programming language, library, application programming interface (API), and/or other software development tools or frameworks. Similarly, schematic elements used to represent data or information may be implemented using any suitable electronic arrangement or data structure. Further, some connections, relationships or associations between elements may be simplified or not shown in the drawings so as not to obscure the disclosure. This disclosure is to be considered as exemplary and not restrictive in character.

## Claims

1. A person support apparatus control system comprising one or more computing devices configured to:
determine a patient lucidity state based on a sensor input;
determine a current angular position of a support section of a person support apparatus; and
cause the support section of the person support apparatus to move from the current angular position to a new angular position in response to the patient lucidity state.

2. The control system of claim 1, wherein the sensor input comprises data indicative of the patient's blood pressure, and the control system is configured to compute the patient lucidity state based on the data indicative of the patient's blood pressure and cause the support section of the person support apparatus to move from the current angular position to the new angular position in response to the patient lucidity state computed based on the data indicative of the patient's blood pressure.

3. The control system of claim 1 or claim 2, wherein the sensor input comprises data indicative of the patient's blood oxygen saturation level, and the control system is configured to compute the patient lucidity state based on the data indicative of the patient's blood oxygen saturation level and cause the support section of the person support apparatus to move from the current angular position to the new angular position in response to the patient lucidity state computed based on the data indicative of the patient's blood oxygen saturation level.

4. The control system of any preceding claim, wherein the sensor input comprises data indicative of the patient's heart rate, and the control system is configured to compute the patient lucidity state based on the data indicative of the heart rate and cause the support section of the person support apparatus to move from the current angular position to the new angular position in response to the patient lucidity state computed based on the data indicative of the patient's heart rate.

5. The control system of any preceding claim, wherein the sensor input comprises data indicative of at least two of: the patient's heart rate, the patient's blood oxygen saturation level, and the patient's blood pressure; the control system is configured to compute the patient lucidity state based on a combination of at least two of the patient's heart rate, the patient's blood oxygen saturation level, and the patient's blood pressure; and the control system is configured to cause the support section of the person support apparatus to move from the current angular position to the new angular position in response to the patient lucidity state computed based on the data indicative of at least two of the patient's heart rate, the patient's blood oxygen saturation level, and the patient's blood pressure.

6. The control system of any preceding claim, configured to compare the patient lucidity state to a previously-determined patient lucidity state and move the support section of the patient support apparatus to an inclined position if the comparison of the patient lucidity state to the previously-determined patient lucidity state is indicative of an increase in the patient's lucidity.

7. The control system of any preceding claim, configured to compare the patient lucidity state to a previously-determined patient lucidity state and move the support section of the patient support apparatus toward a flat position if the comparison of the patient lucidity state to the previously-determined patient lucidity state is indicative of a decrease in the patient's lucidity.

8. A person support apparatus having articulating head and foot sections and the control system of any preceding claim, the patient support apparatus configured to:
raise the head section in response to patient lucidity data indicative of an increase in patient lucidity; and
lower the head section in response to patient lucidity data indicative of a decrease in patient lucidity.

9. The person support apparatus of claim 8, configured to lower the foot section in response to the patient lucidity data indicative of an increase in patient lucidity.

10. The person support apparatus of claim 9, configured to cooperatively lower the foot section and raise the head section in response to the patient lucidity data indicative of an increase in patient lucidity.

11. The person support apparatus of any of claims 8-10, configured to raise the foot section in response to the patient lucidity data indicative of a decrease in patient lucidity.

12. A method for adjusting the position of a person support apparatus having at least a head section and a foot section, in response to changes in patient state, the method comprising, with one or more computing devices, over time:
obtaining a plurality of patient physiological inputs;
determining a patient state based on at least two of the patient physiological inputs;
detecting changes in the patient state; and
progressively adjusting the angular position of the head and foot sections of the person support apparatus in accordance with the detected changes in the patient state.

13. The method of claim 12, comprising progressively moving the head and foot sections to a bed position as the patient state declines, and progressively moving the head and foot sections to a chair position as the patient state improves.

14. The method of claim 12 or claim 13, comprising generating an alert indicative of a change in the patient state, and transmitting the alert to another device.

15. The method of any of claims 12 to 14, comprising sending data indicative of the patient state to a medical record database.
